# EUROPÄISCHE PATENTANMELDUNG

(11) **EP 3 037 070 A1**
(43) Veröffentlichungstag der Anmeldung: **29.06.2016**
(21) Anmeldenummer: 16153916.8
(22) Anmeldetag: 13.11.2012
(51) Int. Cl.: A61F 2/50

(54) **PROTHESENKOSMETIK**

(30) Priorität: 30.11.2011 DE 102011120661
(62) Teilanmeldung aus: 12786851.1
(71) Anmelder: Otto Bock HealthCare GmbH, 37115 Duderstadt (DE)
(72) Erfinder: Sawatzki, Steffen, 99759 Sollstedt (DE); Schneegans, Markus, 37434 Rollshausen (DE); Zarling, Sven, 37115 Duderstadt (DE); Niederstrasser, Sandra, 37115 Duderstadt (DE); Kruse, Andreas, 37339 Worbis (DE); Schlesiger, Ilka, 07745 Jena (DE); Thomas, Christian, 07743 Jena (DE); Haase, Dirk, 07743 Jena (DE)
(74) Vertreter: Stornebel, Kai

(57) **Zusammenfassung**

Die Erfindung betrifft eine Prothesenkosmetik (1) zur Verkleidung einer Prothese (10), mit einem distalen Abschnitt (2) und einem Gelenkabschnitt (3), der im angelegten Zustand der Prothesenkosmetik (1) eine Gelenkeinrichtung (30) abdeckt, die zwischen einer proximalen Prothesenkomponente (40) und einer distalen Prothesenkomponente (20) angeordnet ist, wobei der Gelenkabschnitt (3) aus einem 3D-Abstandsgewirke ausgebildet ist, das ein Obertextil und ein Untertextil aufweist, die durch Stützfäden zueinander beabstandet aneinander festgelegt sind.

## Beschreibung

Die Erfindung betrifft eine Prothesenkosmetik zur Verkleidung einer Prothese, mit einem distalen Abschnitt und einem Gelenkabschnitt, der im angelegten Zustand der Prothesenkosmetik eine Gelenkeinrichtung abdeckt, die zwischen einer proximalen Prothesenkomponente und einer distalen Prothesenkomponente angeordnet ist. Eine solche Prothesenkosmetik ist insbesondere zur Verkleidung von Prothesenbeinen vorgesehen, kann aber auch bei Unterarmprothesen eingesetzt werden, bei denen ein Ellenbogengelenk verkleidet werden muss.

Patienten, die eine prothetische Versorgung benötigen, wünschen sich häufig, dass die prothetische Versorgung möglichst unauffällig bleibt, wobei die Unauffälligkeit sowohl auf die Funktion als auch auf die optische Erscheinung bezogen ist. Besteht beispielsweise die Notwendigkeit, Teile eines Beins oberhalb des Knies zu ersetzen, muss ein Prothesenkniegelenk vorgesehen sein, um die Funktionalität des natürlichen Kniegelenkes zu ersetzen. Dieses Prothesenkniegelenk wird üblicherweise über einen Oberschenkelschaft, der den Oberschenkelstumpf aufnimmt, an dem Patienten befestigt. Distal an dem Prothesenkniegelenk ist ein Unterschenkel, meist in Rohrform, angeordnet, über den ein Prothesenfuß mit dem Prothesenkniegelenk gekoppelt wird. Um eine an ein natürliches Bein angenäherte Formgebung zu erreichen, werden um das Unterschenkelrohr herum Verkleidungsteile angeordnet, meist aus einem Schaumstoff, die die Unterschenkelmuskulatur nachbilden. Über die gesamte prothetische Versorgung aus Protheseneinrichtung und Verkleidung kann ein Überzug aus einem hautfarbigen Material gezogen werden, so dass eine verhältnismäßig unauffällige prothetische Versorgung auch hinsichtlich der Formgebung erreicht werden kann.

Die US 5,133,775 betrifft eine Verkleidung für ein Kunstglied aus einem Polyurethanschaum, der halbsteif ausgebildet ist. Die Verkleidung erstreckt sich über das gesamte Kunstglied und weist im Bereich des Prothesengelenkes eine Vielzahl von Schlitzen auf, die eine Rhombenform aufweisen und zueinander beabstandet in Reihen und Spalten angeordnet sind. Die Verkleidung ist einteilig ausgebildet.

Die WO 2005/009304 A1 betrifft eine Leichtprothese zum rein optischen Ersetzen fehlender Extremitäten mit einem die fehlende Extremität nachbildenden Formkörper und einer Aufnahme zum Verbinden der Prothese mit dem Stumpf der fehlenden Extremität. Der Formkörper besteht aus einem leichten Kunststoffmaterial und kann ein Gelenk oder mehrere Gelenke zur Nachbildung des Gelenks bzw. der Gelenke der fehlenden Extremität beinhalten. Verstärkungsschichten können an der Innenseite des Formkörpers an der Beuge- und Streckseite der Gelenke angeordnet sein.

Die US 7,090,651 B2 betrifft eine Kompressionsorthese mit einer elastischen, tubusförmigen Struktur, die aus einem Zuschnitt eines Kompositmaterials gefertigt wurde. Das Kompositmaterial weist eine erst Schicht mit einem elastischen, dehnbaren Material mit einer inneren Oberfläche und einer äußeren Oberfläche auf. Eine flexible zweite Schicht mit einer äußeren Oberfläche, die an der inneren Oberfläche der ersten Schicht befestigt ist und einer dritten Schicht, die aus einem Abstandsgewirk mit einer äußeren Oberfläche ausgebildet ist, die an der inneren Oberfläche der zweiten Schicht angeordnet ist sowie einer flexiblen vierten Schicht, deren Außenseite an der Innenseite der dritten Schicht befestigt ist. In der ersten Schicht können eine Vielzahl von Schlitzen eingebracht sein. Die zweite und vierte Schicht sind vorzugsweise aus einem Gestrick ausgebildet. Die dritte Schicht, die als Abstandsgewirk ausgebildet ist, weist zwei voneinander beabstandete Oberflächen auf, die auch als Grundgewebe bezeichnet werden, die miteinander durch eine Vielzahl von Abstandfäden miteinander verbunden sind. Ziel der Bandage ist es, eine verbesserte Kompression bereitzustellen, die Körperwärme zurückzuhalten und eine verbesserte Atmungsaktivität zu erreichen.

Die US 7,762,973 B2 beschreibt ein Abstandselement zur verbesserten Anpassung einer Ortheseneinrichtung an den Nutzer. Das Abstandselement kann ein Textil sein, das mit einer Beschichtung versehen ist. Das Abstandselement ist zwischen dem Rahmen der Orthese und dem Orthesennutzer angeordnet.

Die DE 203 09 318 U1 betrifft einen Kniebereichsteilungsadapter zur lösbaren Verbindung eines ersten kosmetischen Schaumteils, das über ein Kniegelenk einer Beinprothese schiebbar und über einen Oberschenkelstumpf stülpbar ist, mit einem zweiten kosmetischen Schaumteil, in das ein Unterschenkelteil der Beinprothese einbindbar ist. Zwei plattenförmige Adapterteile sind vorgesehen, wobei ein erstes Adapterteil mit dem ersten kosmetischen Schaumteil und ein zweites Adapterteil mit dem zweiten kosmetischen Schaumteil lösbar verbindbar sind.

Die DE 100 40 955 A1 beschreibt eine Manschette zum Einhüllen eines Bereichs eines künstlichen Kniegelenkes einer Beinprothese. Die Manschette besteht im Wesentlichen aus einem ringförmigen Grundkörper aus einem flexiblen Material. Das eine Ende des Grundkörpers ist über den Oberschenkelstumpf stülpbar, das andere Ende ist über die Unterschenkelprothese der Beinprothese stülpbar. Im Bereich des Kniegelenkes ist die Manschette als Faltenbalg ausgebildet. Die Manschette bildet gegenüber dem Umfeld ein abdichtendes Element gegen Schmutz und Wasser.

Problematisch bei den meisten Prothesenkosmetiken ist die hohe Belastung im Gelenkbereich aufgrund der großen Deformationen bei einer vollständigen Beugung, verbunden mit hohen Stauchungs- und Dehnungsumfängen. So kommt es insbesondere bei einer Prothesenkosmetik für eine Beinprothese aus einem Schaumstoff oder Gummimaterial zu Rissbildungen. Eine Materialverstärkung an den besonders belasteten Zonen führt nicht zu einer verbesserten Haltbarkeit, zudem beeinflussen dicke Kosmetiken das Beuge- und Streckverhalten der Prothesengelenke.

Aufgabe der vorliegenden Erfindung ist es daher, eine Prothesenkosmetik bereitzustellen, in die keine oder nur eine geringe Funktionsbeeinflussung der Prothese bewirkt und darüber hinaus eine größere Haltbarkeit gewährleistet. Erfindungsgemäß wird diese Aufgabe durch eine Prothesenkosmetik mit den Merkmalen des Hauptanspruches gelöst. Vorteilhafte Ausgestaltungen und Weiterbildungen der Erfindung sind in den Unteransprüchen, der Beschreibung und in den Figuren offenbart.

Die erfindungsgemäße Prothesenkosmetik zur Verkleidung einer Prothese mit einem distalen Abschnitt und einem Gelenkabschnitt, der im angelegten Zustand der Prothesenkosmetik eine Gelenkeinrichtung abdeckt, die zwischen einer proximalen Prothesenkomponente und einer distalen Prothesenkomponente angeordnet ist, sieht vor, dass der Gelenkabschnitt aus einem 3D-Abstandsgewirk ausgebildet ist, das ein Obertextil und ein Untertextil aufweist, die durch Stützfäden zueinander beabstandet aneinander festgelegt sind.

Durch die Ausgestaltung des Gelenkbereiches in der Prothesenkosmetik aus einem 3D-Abstandsgewirk ist es möglich, notwendige Dehnungen und Stauchungen der Prothesenkosmetik im Bereich der Gelenkeinrichtungen, beispielsweise im Bereich des Knies oder des Ellenbogens, zuzulassen, ohne dass eine Funktionsbeeinflussung auf das Prothesenkniegelenk ausgeübt wird. 3D-Abstandsgewirke mit einem Obertextil und einem Untertextil ermöglichen einen hohen Verformungsgrad, ohne dass es zu der Verformung großer Kräfte bedarf. Dadurch wird die Funktion der Prothese, die regelmäßig ohne angelegte Prothesenkosmetik eingestellt und an den Patienten angepasst wird, nicht oder nur minimal beeinflusst. Darüber hinaus wird bei einem geringen Gewicht ein großes Volumen durch das Abstandsgewirk ausgefüllt, wobei eine gute Formstabilität bei gleichzeitiger großer Elastizität erreicht wird. Als Ober- und Untertextil werden Gestricke, Gewebe, Gelege und Gewirke angesehen, die aus einem Verbund von Fasern bestehen.

Der Gelenkabschnitt kann an dem distalen Abschnitt befestigt sein, so dass ein modularer Aufbau der Prothesenkosmetik ermöglicht wird. Der distale Abschnitt verkleidet beispielsweise das Unterschenkelrohr oder die Unterschenkelprothese und gegebenenfalls Teile des Prothesenfußes, während sich der Gelenkabschnitt an das proximale Ende des distalen Abschnittes anschließt. Der Gelenkabschnitt kann mit seinem proximalen Ende direkt an einer proximalen Prothesenkomponente befestigt oder daran anliegend ausgestaltet sein, beispielsweise kann der Gelenkabschnitt an einem Oberschenkelschaft oder einem Oberarmschaft unmittelbar befestigt werden, insbesondere wenn der Oberarmschaft oder Oberschenkelschaft eine entsprechende Kontur aufweist, so dass ein nahezu absatzfreier Übergang erzeugt werden kann.

Der Gelenkabschnitt kann an dem distalen Abschnitt angeklebt, angeschweißt, angenäht, angeklettet oder angeschäumt sein, so dass eine stoffschlüssige oder formschlüssige Verbindung zwischen dem Gelenkabschnitt und dem distalen Abschnitt hergestellt werden kann. Der Gelenkabschnitt kann an der proximalen Prothesenkomponente durch Formschlusselemente oder durch Ankleben oder dergleichen festgelegt werden. Ebenfalls ist es möglich, den Gelenkabschnitt über Magnethalter oder klemmend mit Kraftschluss an dem distalen und/oder proximalen Abschnitten zu befestigen.

Der distale Abschnitt kann aus einem Schaummaterial ausgebildet sein, der als vorgeformter Block von einem Orthopädietechniker an die Protheseneinrichtung angepasst und beispielsweise nach dem Vorbild des unversorgten Beines moduliert wird, so dass eine symmetrische Erscheinung erzielt werden kann. Alternativ zu einem Schaummaterial kann der distale Abschnitt auch aus einem dünnen, glattwandigen Kunststoffmaterial hergestellt sein, dass über geeignete Abstandselemente und Befestigungseinrichtungen an der distalen Prothesenkomponente festgelegt werden kann.

Der Gelenkabschnitt kann einen geschlossenen Querschnitt aufweisen, so dass er als schlauchförmiges Bauteil ausgebildet sein kann. Der geschlossene Querschnitt kann durch ein entsprechendes Herstellverfahren des 3D-Abstandsgewirkes erzeugt werden, alternativ ist ein Vernähen, Verkleben oder Verschweißen eines ebenen Zuschnitts des 3D-Abstandsgewirks möglich und vorgesehen. Alternativ zu einer dauerhaften, geschlossenen Querschnittsform können an dem Gelenkabschnitt auch lösbare Verschlusseinrichtungen vorgesehen sein, beispielsweise Klettverschlüsse, Haken oder dergleichen, so dass auch eine individuelle Anpassung des Umfanges des Gelenkabschnittes einfach möglich ist.

Der distale Abschnitt der Prothesenkosmetik kann über einen Teil seiner Längserstreckung oder über seine gesamte Längserstreckung einen offenen Querschnitt aufweisen, so dass der distale Abschnitt aufgebogen und über die distale Prothesenkomponente aufgestülpt werden kann. Innerhalb der Prothesenkosmetik ist ein Hohlraum zur Aufnahme der Protheseneinrichtung ausgebildet. Der Hohlraum kann sowohl in dem Gelenkabschnitt als auch in dem distalen Abschnitt ausgebildet sein. In dem Gelenkabschnitt kann eine Ausnehmung ausgebildet sein, durch die ein Zugang von außen zu der abgedeckten und geschützt liegenden Gelenkeinrichtung hergestellt werden kann. Dadurch ist es möglich, ohne die Prothesenkosmetik abzulegen, die Protheseneinrichtung einzustellen.

Die Prothesenkosmetik kann darüber hinaus mit einem Überzug aus einem elastischen Material versehen sein, um insbesondere eine glatte Oberfläche zu erzielen, die der Anmutung eines natürlichen Beines ähnelt. Der elastische Überzug kann aus einem Textil oder einem Schaumstoff bestehen und bewirkt aufgrund der hohen Elastizität keine oder nur eine minimale Funktionsbeeinträchtigung.

Der distale Abschnitt weist eine an die Formgebung der natürlichen Gliedmaße angepasste Kontur auf. Die Prothesenkosmetik kann als Verkleidung für ein Prothesenbein ausgebildet und einen vorgeformten Patellabereich oder einen Patellaeinsatz aufweisen, so dass sowohl bei einem gestreckten Prothesenbein als auch bei einem gebeugten Prothesenbein eine der natürlichen Erscheinungsform angenährte Formgestaltung erreicht wird. Der Patellaeinsatz kann aus einem 3D-Abstandsgewirk oder einem anderen Material bestehen.

Neben einem distalen Abschnitt kann auch ein proximaler Abschnitt vorgesehen sein, der an dem Gelenkabschnitt befestigt sein kann. Der proximale Abschnitt der Prothesenkosmetik schließt sich an das proximale Ende des Gelenkabschnittes an und kann beispielsweise über einen Oberarm- oder Oberschenkelschaft gezogen werden, um dort eine glatte Oberfläche oder eine Angleichung an die Form des nicht versorgten, gegenüberliegenden Oberschenkels herzustellen. Der proximale Abschnitt kann analog zu dem distalen Abschnitt ausgebildet sein, beispielsweise aus einem Kunststoff oder einem Schaummaterial. Ebenfalls kann der proximale Abschnitt über zumindest Teile seiner Längserstreckung einen offenen Querschnitt aufweisen. In dem proximalen Abschnitt kann beispielsweise eine Ausnehmung in Keilform ausgebildet sein, in den unterschiedlich große Einsätze eingesetzt werden können, um eine Umfangsanpassung des proximalen Abschnitts zu ermöglichen. Der Gelenkabschnitt kann an dem proximalen Abschnitt analog zu dem distalen Abschnitt angeklebt, angeschweißt, angenäht, angeklettet oder angeschäumt werden. Neben einer permanenten Anbindung kann der Gelenkabschnitt an dem proximalen Abschnitt auch lösbar befestigt sein; ebenso ist die Befestigung über Kraftschluss möglich und vorgesehen.

Eine Weiterbildung der Erfindung sieht vor, dass der Gelenkabschnitt an der Gelenkkomponente befestigt sein kann, so dass eine direkte Zuordnung des Gelenkabschnittes zu der Gelenkkomponente erfolgen kann. Es ist damit grundsätzlich möglich, dass die Verkleidungsabschnitte der Prothesenkosmetik separat an den Prothesenkomponenten befestigt sind, ohne dass sie miteinander verbunden sind. Es besteht aber auch die Möglichkeit, dass der Gelenkabschnitt an dem distalen Abschnitt und/oder dem proximalen Abschnitt befestigt ist, wobei diese Abschnitte ebenfalls an den proximalen und distalen Prothesenkomponenten befestigt sein können.

Nachfolgend werden Ausführungsbeispiele der Erfindung anhand der beigefügten Figuren näher erläutert. Es zeigen:
- Figur 1 -: eine schematische Ansicht einer Prothesenkosmetik im angelegten Zustand mit einem Überzug;
- Figur 2 -: eine Ausgestaltung gemäß Figur 1 ohne Überzug;
- Figur 3 -: eine Detailansicht eines Gelenkbereiches;
- Figur 4 -: einen Gelenkbereich mit einem Patellaeinsatz;
- Figur 5 -: eine perspektivische Detailansicht einer Variante eines Gelenkbereiches;
- Figur 6 -: eine Einzelansicht eines distalen Abschnitts;
- Figur 7 -: eine Detailansicht eines proximalen Abschnittes;
- Figur 8 -: eine Einzeldarstellung eines Gelenkbereiches;
- Figur 9 -: eine Einzeldarstellung eines Verbindungselementes zwischen einem Gelenkbereich und einem proximalen Abschnitt;
- Figur 10-: eine alternative Befestigungsmöglichkeit des Gelenkabschnittes an einem proximalen oder distalen Abschnitt;
- Figur 11 -: eine Darstellung einer Volumenanpassung; sowie
- Figur 12 -: eine Variante der Figur 11.

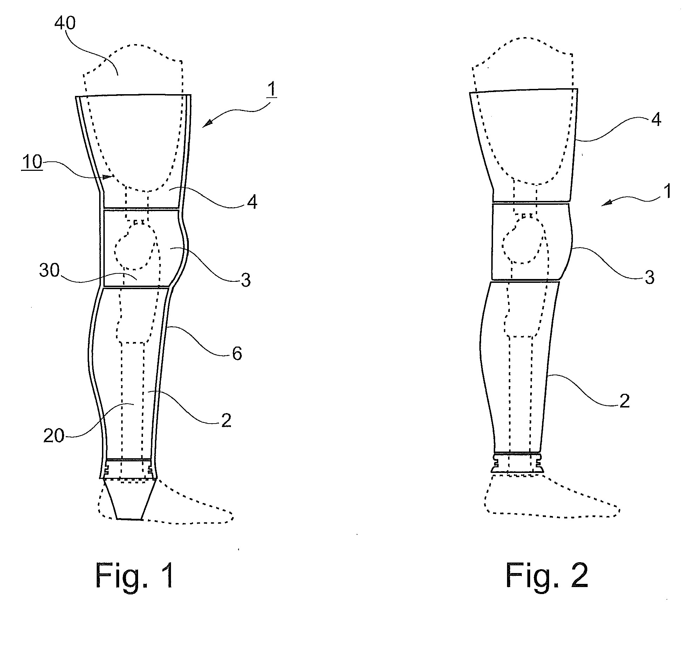

In der Figur 1 ist eine Prothesenkosmetik 1 in einem angelegten Zustand an einer Prothese 10, die als ein Prothesenbein ausgebildet ist, dargestellt. Die Prothese 10 weist eine distale Komponente 20 in Gestalt eines Unterschenkelrohrs mit einem daran befestigten Prothesenfuß, ein Prothesenkniegelenk 30 sowie eine proximale Prothesenkomponente 40 in Gestalt eines Oberschenkelschaftes auf. Die proximale Komponente 40 ist über das Prothesenkniegelenk 30 mit der distalen Komponente 20 gelenkig verbunden.

Um die Prothese 10 ist die Prothesenkosmetik 1 herum angelegt, die formbildende Komponenten aufweist. Um die distale Prothesenkomponente 20 ist ein distaler Abschnitt 2 angeordnet, der die äußere Form einer natürlichen Wade aufweist. Oberhalb der distalen Abschnitts 2, also proximal dazu, ist ein Gelenkabschnitt 3 angeordnet, der sich im Bereich eines natürlichen Kniegelenkes befindet und um die nicht eingezeichnete Schwenkachse des Prothesenkniegelenkes 30 herum angeordnet ist. Der Gelenkabschnitt 3 umhüllt den Bereich um die Gelenkachse des Prothesenkniegelenkes 30 vollständig und erstreckt sich proximal und distal der Gelenkachse. Proximal zu dem Gelenkabschnitt 3 ist der proximale Abschnitt 4 angeordnet, der den Übergang von dem Gelenkbereich 3 zu dem Oberschenkelschaft 40 ausformt. Der proximale Abschnitt 4 bildet die Kontur eines natürlichen Oberschenkels ab und dient dazu, einen möglichst unauffälligen Übergang von dem Oberschenkelschaft 40 zu dem Gelenkabschnitt 3 zu erzielen. Die übereinander angeordneten Abschnitte 2, 3, 4 der Prothesenkosmetik 1 bilden insgesamt eine angenäherte äußere Kontur eines natürlichen Beines ab. Auf der Außenseite der Abschnitte 2, 3, 4 ist ein Überzug 6 aus einem hochelastischen Material, beispielsweise einem hochelastischen Textil, angeordnet, um eine geschlossene Oberfläche für einen harmonischen Gesamteindruck zu erzielen und darüber hinaus Übergänge zwischen den einzelnen Abschnitten 2, 3, 4 zu kaschieren.

Der Gelenkbereich 3 ist aus einem 3D-Abstandsgewirk ausgebildet und weist ein Obertextil, ein Untertextil und Stützfäden auf, die das Obertextil und das Untertextil beabstandet zueinander aneinander befestigen. Die Stützfäden können aus einem anderen Material als das Obertextil und das Untertextil ausgebildet sein, ebenfalls können Obertextil aus unterschiedlichen Materialien hergestellt sein, um eine Anpassung an die gewünschten Eigenschaften zu ermöglichen.

In der Figur 2 ist die Prothesenkosmetik 1 ohne den Überzug 6 dargestellt. Die einzelnen Abschnitt 2, 3, 4 sind als modulare Komponenten ausgebildet und können einzeln an der Prothese 10 festgelegt werden. Unterhalb des distalen Abschnitts 2 ist ein Überbrückungselement zur Anbindung an den Prothesenfuß dargestellt.

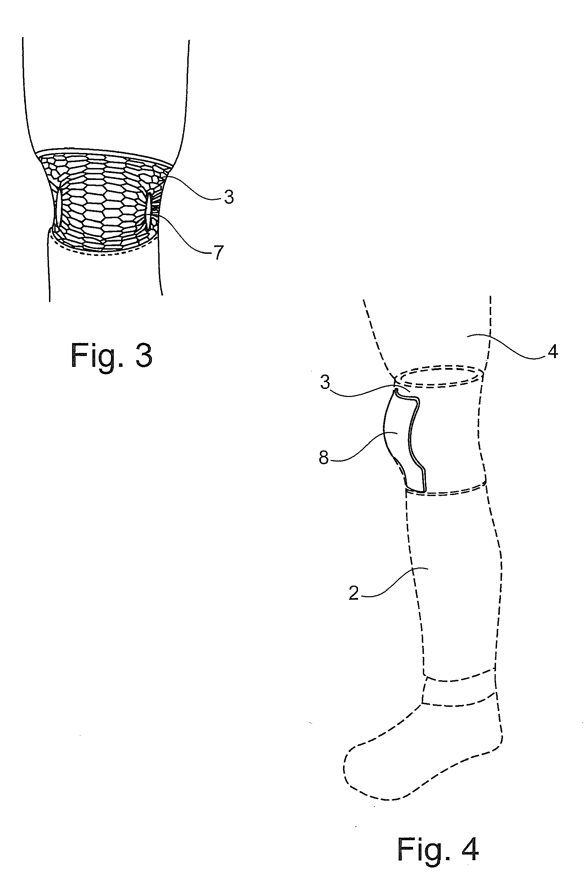

Der Gelenkabschnitt 3 aus dem 3D-Abstandsgewirk weist in der dargestellten gestreckten Stellung frontal eine Pantellaauswölbung auf, die der Patella eines natürlichen Beines angenähert ausgebildet ist. Der Gelenkabschnitt 3 ist rohrförmig ausgebildet und ist mit seinem distalen Ende und seinem proximalen Ende mit den jeweils angrenzenden Abschnitten 2, 3 verbunden. In der Figur 3 ist in einer Einzeldarstellung eine Frontalansicht des Gelenkabschnittes 3 dargestellt, anhand der die wabenartige Struktur des Obertextils des 3D-Abstandsgewirkes erkannt werden kann. Ein vorgeformter Patellabereich 7 wird durch Verkürzen seitlicher Bereiche des Gelenkabschnittes 3 ausgebildet, so dass sich Einschnürungen beiderseits einer nicht dargestellten senkrecht verlaufenden Mittelebene ergeben, die eine an die natürliche Form angenäherte Gestalt des Gelenkbereiches 3 bewirken. Die Einschnürungen 7 können durch Nähte oder dergleichen ausgebildet werden.

In der Figur 4 ist eine Variante der Erfindung dargestellt, bei der der distale Abschnitt 2 und der proximale Abschnitt 4 nur angedeutet sind. Im Gelenkabschnitt 3 ist ein Patellaeinsatz 8 aus einem elastischen, flexiblen Material eingesetzt, beispielsweise einem 3D-Abstandsgewirk, das aufgrund einer Beschichtung, dem Einsatz anderer Materialien oder anderer Fadenanordnungen eine gegenüber dem übrigen 3D-Abstandsgewirk erhöhte Festigkeit und Formstabilität aufweist, so dass zwar eine Beugung des Prothesenkniegelenkes ohne Weiteres möglich ist, die Form der Patella im frontalen Bereich jedoch angedeutet bleibt. Alternativ kann der Patellaeinsatz 8 als Einsatz in Form eines Löffels ausgebildet sein, der am distalen Abschnitt 2 befestigt ist. Der Einsatz befindet sich hinter dem 3D-Abstandsgewirk und bildet eine Patalla aus. Vorzugsweise federt der Einsatz bei Beugung und Streckung elastisch nach und bildet durch die Anlagekraft die Patella nach.

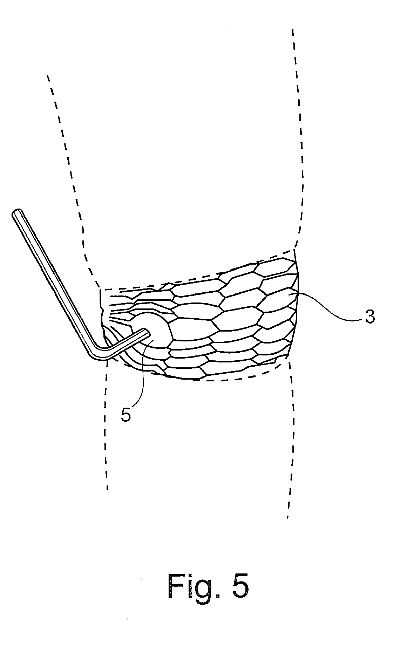

In der Figur 5 ist eine perspektivische Ansicht des Gelenkabschnittes 3 mit einer Öffnung 5 im medialen oder lateralen Bereich dargestellt. Durch die Ausnehmung 5 kann ein Werkzeug eingeführt werden, über das das Prothesengelenk 30 eingestellt und an den jeweiligen Nutzer angepasst werden kann.

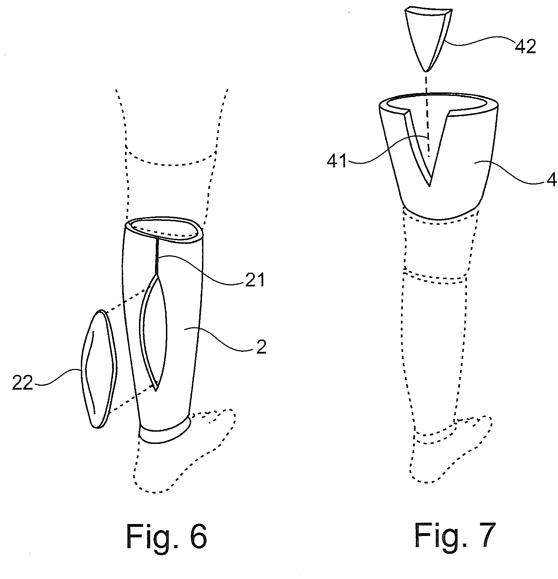

In der Figur 6 ist eine perspektivische Rückenansicht des distalen Abschnittes 2 dargestellt. Es ist zu erkennen, dass ein Schlitz 21 sich über eine Teillänge des distalen Abschnittes 2 erstreckt, so dass ein teilweise offener Hohlquerschnitt gebildet wird. Im rückwärtigen Bereich kann ein Einsatz 22 eingebracht werden, um unterschiedliche Umfänge des distalen Abschnittes 2 auszubilden, so dass auch bei einer Ausgestaltung des distalen Abschnittes 2 aus einem dünnwandigen Material, beispielsweise einem dünnwandigen Schaumstoff oder einem elastischen Kunststoff, eine erleichterte Anpassung an unterschiedliche Formgestaltungen erreicht werden kann.

Figur 7 zeigt eine Einzeldarstellung des proximalen Abschnittes 4 mit einer keilförmigen Ausnehmung 41 im rückwärtigen Bereich, die dazu ausgebildet ist, um einen Einsatz 42 aufzunehmen. Auch hier ist ein teilweise offener Querschnitt über die Längserstreckung des proximalen Abschnittes 4 ausgebildet. Über die Einsätze 42 kann ebenfalls eine Anpassung an den jeweils gewünschten Umfang erreicht werden. Neben den in den Figuren 6 und 7 dargestellten Ausführungsbeispielen für eine Verkleidung eines Prothesenbeines, kann die Volumenanpassung der Kosmetiken durch Einsätze auch bei anderen Protheseneinrichtungen erfolgen, beispielsweise bei Armprothesen. Auch hier können durch das gezielte Einbringen von Ausnehmungen und das Auffüllen mit Einsätzen unterschiedlicher Größe unterschiedliche Kosmetikvolumina realisiert werden. Je größer die Einsätze bei gleich bleibender Größe der Ausnehmungen, desto größer wird das Volumen der angepassten Prothesenkosmetik, wobei der Einsatz mit der übrigen Prothesenkosmetik vorzugsweise bündig abschließt und die Materialstärke der Einsätze der Materialstärke der übrigen Prothesenkosmetik im Wesentlichen entspricht.

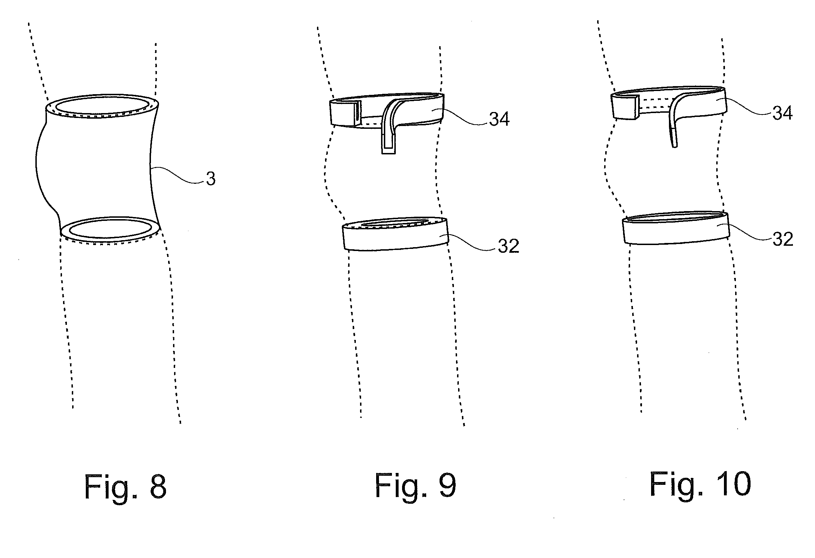

In der Figur 8 ist eine Einzeldarstellung des Gelenkabschnittes 3 gezeigt. Die hohlkörperförmige, tubusartige Ausgestaltung des Gelenkabschnittes 3 mit einem geschlossenen Querschnitt ist zu erkennen. Ebenfalls ist die Patellaauswölbung im vorderen Bereich zu sehen. Der röhrenförmige Gelenkabschnitt 3 weist eine Materialstärke auf, die im Wesentlichen der Materialstärke der distalen und proximalen Abschnitte 2, 4 im angrenzenden Bereich entspricht, so dass eine leichte Zuordnung der jeweiligen Abschnitte 2, 3, 4 zueinander erfolgen kann. Im Inneren des Gelenkabschnittes 3 ist ein ausreichend großer Hohlraum vorhanden, um eine freie Beweglichkeit des Prothesengelenks zu ermöglichen.

In der Figur 9 ist eine Befestigungsmöglichkeit des Gelenkabschnittes 3 an dem distalen Abschnitt 2 und dem proximalen Abschnitt 4 gezeigt. Zwei Verbindungsbänder 32, 34 sind an dem Gelenkabschnitt 3 befestigt und weisen eine Nut und eine Ausnehmung auf, in die der jeweils angrenzende Abschnitt 2, 4 einführbar ist. In der Nut kann der jeweilige Abschnitt 2, 4 eingeklebt, damit vernäht, eingeklettet, eingeklemmt oder darin eingeschweißt werden.

Eine alternative Befestigung ist in der Figur 10 gezeigt, bei der die Befestigungsbänder 32, 34 lediglich an dem Außenumfang der jeweiligen Abschnitte 2, 3, 4 angeordnet und befestigt sind. Die Befestigungsbäder 32, 34 können als Klebebänder, Klettbänder oder einer Kombination davon ausgebildet sein.

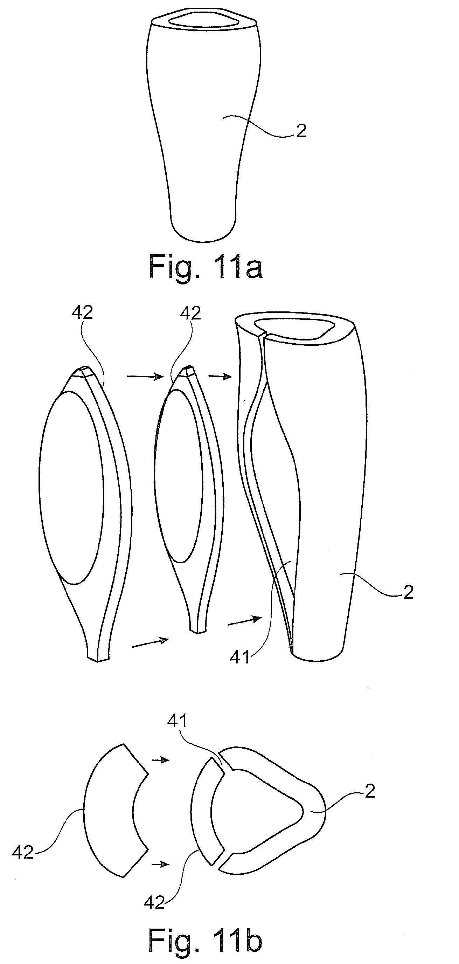

In der Figur 11a ist ein distaler Abschnitt 2 in einer perspektivischen Darstellung gezeigt. Der distale Abschnitt 2 ist als Grundkörper ausgebildet, der vorgefertigt angeliefert wird. Der distale Abschnitt 2 ist als Hohlkörper ausgebildet, in dessen Hohlraum ein Unterschenkelrohr zur Verbindung eines Prothesenkniegelenkes mit einem Prothesenfuß untergebracht werden kann. Weitere Komponenten der prothetischen Versorgung können ebenfalls in dem Hohlraum angeordnet sein. Die äußere Form des distalen Abschnittes 2 entspricht der eines natürlichen Unterschenkels, wobei bevorzugt eine Dimensionierung gewählt ist, die einen möglichst großen Bereich an Variationen abdeckt. Es können auch mehrere Grundkörper des distalen Abschnitts 2 in verschiedenen Größen angeboten werden, so dass für kleine, mittlere und große Patienten mit unterschiedlichen Wadendurchmessern korrespondierende distale Abschnitte 2 angefertigt und angepasst werden können. Als ersten Schritt zur Anpassung wird eine Einstellung der Länge des distalen Abschnitts 2 vorgenommen. Ein Orthopädietechniker kann beispielsweise am distalen Ende des distalen Abschnitts 2 eine entsprechende Kürzung vornehmen, wie sie durch die gestrichelte Linie angedeutet ist, so dass der distale Abschnitt 2 an die Erfordernisse angepasst ist.

Nach der Längenanpassung, wie sie in der Figur 11a dargestellt ist, kann eine Volumenanpassung erfolgen. Dies geschieht, indem aus dem distalen Abschnitt 2 Material herausgeschnitten wird, so dass eine Ausnehmung 41 entsteht. In dem dargestellten Ausführungsbeispiel wird zuerst ein Längsschlitz in den distalen Abschnitt 2 über dessen gesamte Länge eingebracht, so dass sich insgesamt ein offener Querschnitt bildet. Der Längsschnitt wird anschließend geweitet, so dass eine linsenförmige oder tropfenförmige Ausnehmung 41 in dem distalen Abschnitt 2, der aus einem Schaumstoff in einem anderen Kunststoff ausgebildet sein kann, ausgebildet wird. Die Aufweitung geschieht in der Regel durch eine Materialentfernung und durch ein Aufbiegen des Grundkörpers. In diese Ausnehmung 41 wird dann ein entsprechend konturierter Einsatz 42 eingesetzt und entlang der Seitenkanten der Ausnehmung 41 verbunden, beispielsweise verklebt oder verschweißt. In der oberen Darstellung der Figur 11b sind mehrere Einsätze 42 gezeigt, die jeweils ein unterschiedliches Volumen haben. Durch den Einsatz eines größeren Einsatzes 42, in der Figur 11b der linke Einsatz 42, kann ein größeres Volumen des distalen Abschnitts 2 realisiert werden, soll nur eine geringere Volumenvergrößerung des distalen Abschnitts 2 erfolgen, wird der rechte Einsatz 42 in die Ausnehmung 41 eingesetzt und mit dem übrigen Material verbunden.

Die untere Darstellung der Figur 11b zeigt eine Querschnittsansicht, wobei der Querschnitt ungefähr auf der Mitte des distalen Abschnittes 2 gelegt ist. Neben dem distalen Abschnitt 2 und dem Hohlraum ist die Ausnehmung 41 zu erkennen, in die der Einsatz 42 eingesetzt ist. Eine Verklebung oder ein Verschweißen des Einsatzes 42 mit dem übrigen distalen Abschnitt 2 hat noch nicht stattgefunden. Wie der Querschnittsdarstellung zu entnehmen ist, ist der linke Einsatz 42 nicht nur hinsichtlich des Umfanges größer, sondern auch des Volumens, das heißt, dass der Ansatz 42 eine größere Wandstärke aufweist. Durch die individuelle Ausgestaltung der Einsätze 42 ist es möglich, aus einem vorgeformten Kontingent an Einsätzen 42 auszuwählen und eine Ausgestaltung der distalen Abschnitte 2 zu erreichen, die den Formen des kontralateralen, also unversorgten Beines entspricht.

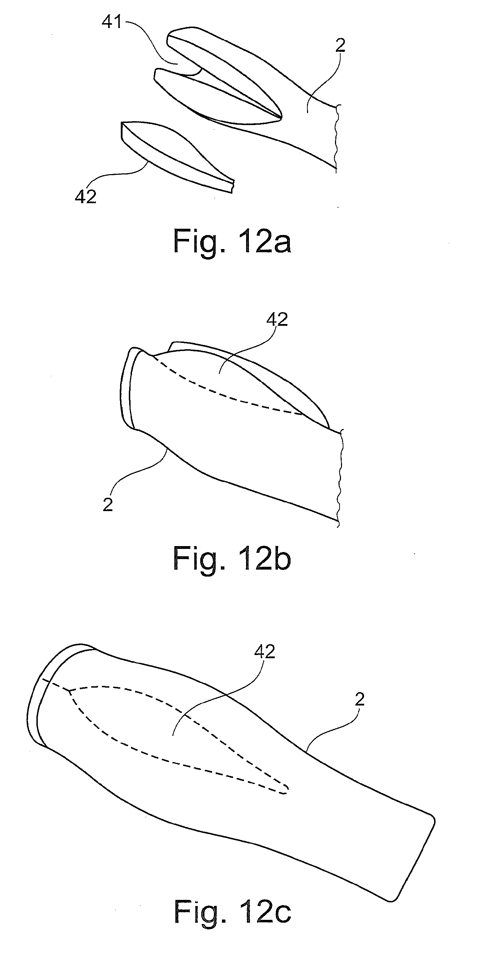

Eine Variante der Volumenanpassung ist in den Figuren 12a bis 12c dargestellt. In der Figur 12a ist der distale Abschnitt mit der Ausnehmung 41 in Gestalt eines Einschnittes ausgebildet, der sich nicht über die gesamte Längserstreckung des distalen Abschnitts 2 erstreckt. Der Einsatz 42, der zur Volumenanpassung in die Ausnehmung 41 eingesetzt werden soll, liegt neben dem distalen Abschnitt 2. In der Figur 12b ist ein Zwischenschritt bei der Volumenanpassung zu sehen, bei dem der Einsatz 42 bereits an einer Seite der Ausnehmung 41 an dem distalen Abschnitt 2 befestigt ist, beispielsweise angeschweißt oder angeklebt. In der Figur 12c ist der fertiggestellte distale Abschnitt 2 zu erkennen, bei dem der Einsatz 42 vollständig in dem distalen Abschnitt 2 eingearbeitet ist. Eventuell vorhandene Überstände sind geglättet, so dass eine glatte, im Wesentlichen kantenlose Oberfläche des distalen Abschnitts 2 realisiert wird.

Die Volumenanpassung gemäß der Ausführungen zu den Figuren 6, 7, 11 und 12 können auch separat vorgenommen werden, ohne dass ein proximaler Abschnitt und/oder ein Gelenkabschnitt an dem distalen Abschnitt 2 befestigt werden muss. Eine solche Vorgehensweise bietet sich an, wenn eine Protheseneinrichtung nicht im Kniebereich verkleidet werden muss oder es sich nur um eine Unterschenkelprothese handelt. Das Gleiche kann auch für andere Extremitäten durchgeführt werden, beispielsweise zur Volumenanpassung von Verkleidungen an Oberschenkeln oder Armprothesen. Durch das vorgestellte Verfahren ist es möglich, eine individualisierbare Prothesenkosmetik mit zusätzlichen Volumenelementen in Gestalt von Einsätzen 42 zur Herstellung einer kosmetischen Verkleidung für Prothesen bereitzustellen. Die einfache Individualisierung auf die gewünschten Patientenabmaße ist durch ein Baukastensystem möglich, das neben Grundkörpern auch unterschiedliche Größen von Einsätzen 42 aufweist, die dann an den jeweiligen Patienten angepasst werden können. Dadurch ist es möglich, dass eine schnellere Anpassung und eine kostengünstigere Herstellung einer kosmetischen Prothesenverkleidung verwirklicht werden kann. Die Einsätze 42 können unterschiedliche Formen aufweisen, vorteilhafterweise weisen sie eine ähnliche Kontur auf, so dass die Ausnehmungen 41 im Wesentlichen dieser Kontur folgen, wobei auf die gewünschte Volumenzunahme Rücksicht genommen wird. Die Einsätze 42 sind ebenso wie die Grundkörper industriell vorfertigbar, so dass die Formteile zur Anpassung der Prothesenkosmetik an den jeweiligen Patienten leicht konfektionierbar sind. Dadurch wird die Herstellung einer kosmetischen Bekleidung für eine Protheseneinrichtung vereinfacht. Die fertiggestellte Prothesenkosmetik kann dann noch mit einem Überzug versehen werden, beispielsweise einem strumpfähnlichen Gewebe, so dass eine gleichmäßige Färbung und hautähnliche Oberflächenstruktur erzielt werden kann.

## Patentansprüche

1. Prothesenkosmetik (1) zur Verkleidung einer Prothese (10), mit einem distalen Abschnitt (2) und einem Gelenkabschnitt (3), der im angelegten Zustand der Prothesenkosmetik (1) eine Gelenkeinrichtung (30) abdeckt, die zwischen einer proximalen Prothesenkomponente (40) und einer distalen Prothesenkomponente (20) angeordnet ist, **dadurch gekennzeichnet, dass** der Gelenkabschnitt (3) aus einem 3D-Abstandsgewirke ausgebildet ist, das ein Obertextil und ein Untertextil aufweist, die durch Stützfäden zueinander beabstandet aneinander festgelegt sind.

2. Prothesenkosmetik nach Anspruch 1, **dadurch gekennzeichnet, dass** der Gelenkabschnitt (3) an dem distalen Abschnitt (2) befestigt ist.

3. Prothesenkosmetik nach Anspruch 1 oder 2, **dadurch gekennzeichnet, dass** der Gelenkabschnitt (3) an dem distalen Abschnitt (2) angeklebt, angeschweißt, angenäht, angeklettet oder eingeschäumt ist.

4. Prothesenkosmetik nach einem der voranstehenden Ansprüche, **dadurch gekennzeichnet, dass** der distale Abschnitt (2) aus einem Schaummaterial ausgebildet ist.

5. Prothesenkosmetik nach einem der voranstehenden Ansprüche, **dadurch gekennzeichnet, dass** der Gelenkabschnitt (3) einen geschlossenen Querschnitt aufweist.

6. Prothesenkosmetik nach einem der voranstehenden Ansprüche, **dadurch gekennzeichnet, dass** der distale Abschnitt (2) einen über zumindest einen Teil der seiner Längserstreckung offenen Querschnitt aufweist.

7. Prothesenkosmetik nach einem der voranstehenden Ansprüche, **dadurch gekennzeichnet, dass** ein Hohlraum zur Aufnahme der Protheseneinrichtung (10) ausgebildet ist.

8. Prothesenkosmetik nach einem der voranstehenden Ansprüche, **dadurch gekennzeichnet, dass** in dem Gelenkabschnitt (3) eine Ausnehmung (5) ausgebildet ist.

9. Prothesenkosmetik nach einem der voranstehenden Ansprüche, **dadurch gekennzeichnet, dass** sie mit einem Überzug (6) aus einem elastischen Material versehen ist.

10. Prothesenkosmetik nach einem der voranstehenden Ansprüche, **dadurch gekennzeichnet, dass** sie als Verkleidung für ein Prothesenbein (10) ausgebildet ist und einen vorgeformten Patellabereich (7) oder einen Patellaeinsatz (8) aufweist.

11. Prothesenkosmetik nach einem der voranstehenden Ansprüche, **dadurch gekennzeichnet, dass** ein proximaler Abschnitt (4) vorgesehen ist, der an dem Gelenkabschnitt (3) befestigt ist.

12. Prothesenkosmetik nach einem der voranstehenden Ansprüche, **dadurch gekennzeichnet, dass** der Gelenkabschnitt (3) an der Gelenkkomponente (30) befestigt ist.
